# EUROPEAN PATENT SPECIFICATION

(11) **EP 4 169 508 B1**
(45) Date of publication and mention of the grant of the patent: **20.05.2026**
(21) Application number: 22187029.8
(22) Date of filing: 01.10.2015
(51) Int. Cl.: A61K 9/20, A61K 31/4439, A61K 31/454, A61P 35/00, A61P 35/02, A61P 43/00, A61J 3/10

(54) **PHARMACEUTICAL COMPOSITIONS COMPRISING ALPELISIB**
PHARMAZEUTISCHE ZUSAMMENSETZUNGEN MIT ALPELISIB
COMPOSITIONS PHARMACEUTIQUES CONTENANT DE L'ALPÉLISIB

(30) Priority: 03.10.2014 US 201462059331 P
(43) Date of publication of application: 26.04.2023
(62) Divisional of application: 15778761.5
(73) Proprietor: Novartis AG, 4056 Basel (CH)
(72) Inventor: Bock, Michaela Anna Maria, 4002 Basel (CH); Elbaz, Frantz, 4056 Basel (CH); Kochhar, Charu, 4002 Basel (CH); Stingelin, Doris, 4002 Basel (CH)
(74) Representative: Carpmaels & Ransford LLP

(56) References cited:
- EP-A1- 2 742 940
- WO-A1-2012/175522
- WO-A1-2013/144249

## Description

### Field of the Invention

The present invention relates to novel dispersible tablets comprising the compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof.

### Background of the Invention

Phosphatidylinositol 3-kinases ("PI-3 kinase" or "PI3K") comprise a widely expressed family of lipid kinases that function as key signal transducers downstream of cell-surface receptors and key regulators of cell metabolism and survival.

Of the two Class I Pl3Ks, Class IA PI3Ks are heterodimers composed of a catalytic p110 subunit (α, β, δ isoforms) constitutively associated with a regulatory subunit that can be p85α, p55α, p50α, p85β or p55y. These catalytic p110 subunits (α, β, δ isoforms) are encoded by three genes (PIK3CA, PIK3CB and PIK3CD) in humans. Class 1B PI3K has one family member, a heterodimer composed of a catalytic p110γ subunit associated with one of two regulatory subunits, either the p101 or the p84. The modular domains of the p85/55/50 subunits include Src Homology (SH2) domains that bind phosphotyrosine residues in a specific sequence context on activated receptor and cytoplasmic tyrosine kinases, resulting in activation and localization of Class IA Pl3Ks. Class IB, as well as p110β in some circumstances, is activated directly by G protein-coupled receptors that bind a diverse repertoire of peptide and non-peptide ligands (Stephens et al., Cell 89:105 (1997)); Katso et al., Annu. Rev. Cell Dev. Biol. 17:615-675 (2001)). Consequently, resultant phospholipid products of class I PI3K link upstream receptors with downstream cellular activities including proliferation, survival, chemotaxis, cellular trafficking, motility, metabolism, inflammatory and allergic responses, transcription and translation (Cantley et al., Cell 64:281 (1991); Escobedo and Williams, Nature 335:85 (1988); Fantl et al., Cell 69:413 (1992)).

Deregulation of PI3K is one of the most common deregulations associated with human cancers and proliferative diseases (Parsons et al., Nature 436:792 (2005); Hennessey at el., Nature Rev. Drug Disc. 4:988-1004 (2005)). The tumor suppressor gene PTEN, which dephosphorylates phosphoinositides at the 3' position of the inositol ring and in so doing antagonizes PI3K activity, is functionally deleted in a variety of tumors. The genes for the p110α isoform (PIK3CA) is amplified and increased protein expression of its gene product has been demonstrated in several human cancers. Mutations and translocation of p85α that serve to up-regulate the p85-p110 complex have been described in human cancers. Finally, somatic missense mutations in PIK3CA that activate downstream signaling pathways have been described at significant frequencies in a wide diversity of human cancers, including 32% of colorectal cancers, 27% of glioblastomas, 25% of gastric cancers, 36% of hepatocellular carcinomas, and 18-40% of breast cancers.

(S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) is a specific 2-carboxamide cycloamino urea derivative compound that potently and selectively targets the alpha (α)-isoform of class IA PI3K. This compound has the following chemical structure: 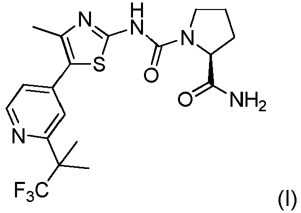 (hereinafter, "Compound I"). Compound I has demonstrated clinical efficacy in the single agent treatment of patients having advanced solid malignancies carrying an alteration in the PIK3CA gene in a Phase I clinical trial. As of March 10, 2014, out of the 131 patients evaluable for radiologic response, 15 patients with PIK3CA-altered solid tumors had partial response to treatment with Compound I and 68 patients had stable disease, as defined by Response Evaluation Criteria in Solid Tumors (RECIST) guideline version 1.0 criteria (Therasse P. et al., "New Guidelines to Evaluate the Response to Treatment in Solid Tumors", JNCI National Cancer Inst. (2000), 2(3): 205-216.). Further, Compound I has demonstrated in vivo dose-dependent antitumor activity in PIK3CA-mutant or amplified tumor xenograft models, such as breast, head and neck, and ovarian cancers. Currently, Compound I is being evaluated in a Phase Ib/II clinical trial in combination with cetuximab for the treatment of patients with recurrent or metastatic head and neck squamous cell carcinoma (NCT01602315).

Depending on age, patient condition, mode of administration, and type of disease, Compound I or a pharmaceutically acceptable salt thereof is orally administered at an effective daily dosage of about 1 to 6.5 mg/kg in adults or children. In a 70 kg body weight adult patient, Compound I or a pharmaceutically acceptable salt thereof is orally administered at a daily dosage of about 70 mg to 455 mg. Compound I or a pharmaceutically acceptable salt thereof is currently orally administered to patients once or twice daily in one or more solid tablets swallowed by said patient. These dosage forms provide efficacious administration of this agent, are convenient to administer, and are stable. However, for certain groups of patients, oral administration of medicaments in solid tablet form is either undesirable or impractical. In particular, children, elderly patients and patients suffering from head and neck cancers may be unable to swallow such tablets conveniently. For these patients, it is typically desirable or necessary to provide alternative dosage forms or alternative methods for administering a medicament.

WO 2013/144249 A1 relates to selective cancer treatment regimes based on assaying for the presence or absence of a mutation in a nucleic acid that encodes glutamine at position 859 of the catalytic p110α subunit of PI3K.

It has been surprisingly found that Compound I or a pharmaceutically acceptable salt thereof may be formulated in form of the novel dispersible tablet of the present invention with high drug loading (e.g., from about 5% to 50%, preferably about 35% to 45%, weight based on the total weight of the tablet) and which disperses in aqueous medium in 5 minutes or less, preferably 3 minutes or less and which produces a smooth dispersion which can pass through a sieve screen with a nominal mesh aperture of 710µm. This novel dispersible tablet also allows Compound I or a pharmaceutically acceptable salt thereof to be conveniently and safely administered to patients with swallowing difficulties at a pharmacologically active daily dosage amount of Compound I.

### Summary of the Invention

The invention is defined in the appended claims.

The present invention provides a dispersible tablet comprising (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of 5% to 50% in weight based on the total weight of the tablet, (b) sodium starch glycolate in an amount of 2% to 12% in weight based on the total weight of the tablet, (c) low-substituted hydroxypropyl cellulose in an amount of 1% to 10 % in weight based on the total weight of the tablet, and (d) sodium stearyl fumarate in an amount of 1% to 7 % in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

Preferably, the dispersible tablet of the present invention comprises granulates having an inner phase and outer phase, wherein the inner phase comprises (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, sodium starch glycolate, low-substituted hydroxypropyl cellulose, and optionally any additional excipients, wherein the outer phase comprises sodium starch glycolate, sodium stearyl fumarate and optionally any additional excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

In one embodiment, the present invention relates to a dispersible tablet comprising (a) *(S)-*Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 35% to 45% in weight based on the total weight of the tablet, (b) sodium starch glycolate in an amount of about 5% to 10% in weight based on the total weight of the tablet, (c) low-substituted hydroxypropyl cellulose in an amount of about 1% to 3% in weight based on the total weight of the tablet, and (d) sodium stearyl fumarate in an amount of about 3% to 5% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

In one embodiment, the dispersible tablets of the present invention is administered to a patient, such as a child or an adult suffering from a proliferative disease (e.g., a cancer), by (i) contacting said tablet with an ingestible liquid, (ii) allowing the tablet to disperse in the ingestible liquid to form a dispersed mixture, e.g., in 5 minutes or less, preferably 3 minutes or less, and (iii) ingesting the dispersed mixture.

In one embodiment, the dispersible tablets of the present invention may be administered to a patient in need thereof by (i) contacting said tablet with an ingestible liquid (ii) allowing the tablet to disperse in the ingestible liquid to form a dispersed mixture, e.g., in 5 minutes or less, preferably 3 minutes or less, and (iii) administering said dispersed mixture said patient using or via a feeding tube, preferably a gastronomy tube (G-tube) or PEG (percutaneous endoscopic gastrostomy) tube.

In one aspect, the present invention relates to a process for producing a dispersible tablet of the present invention, comprising
(a) forming a granulate having an inner phase and an outer phase by
   (i.) wet granulating an inner phase comprising (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of 5% to 50% in weight based on the total weight of the tablet, sodium starch glycolate in a total amount of 1% to 7% in weight based on the total weight of the tablet, low-substituted hydroxypropyl cellulose in a total amount of 1% to 10% in weight based on the total weight of the tablet, and at least one diluent;
   (ii.) adding sodium starch glycolate in a total amount of 1% to 5% in weight based on the total weight of the tablet, and at least one diluent to the inner phase formed in step (a)(i.) and mixing; and
   (iii.) adding sodium stearyl fumarate in a total amount of 1% to 7% in weight based on the total weight of the tablet to the mixture formed in step (a)(ii.) and mixing; and
(b) forming the dispersible tablet by compressing the mixture obtained in step (a)(iii.), with the proviso that the diluent according to step (a)(i.) or (a)(ii.) is not calcium phosphate tribasic, calcium phosphate dibasic anhydrous or calcium phosphate dibasic dihydrate. It is understood that steps (a)(ii) and (a)(iii) form the outer phase of the granulate. In one embodiment, the process further comprises the step of applying a film-coat to the tablet.

In one embodiment, the present invention relates to the dispersible tablets of the present invention for use in the treatment or prevention of a proliferative disorder, preferably a cancer.

In one embodiment, the present invention relates to use of the dispersible tablet of the present invention for the treatment or prevention of a proliferative disease, preferably a cancer.

In one embodiment, the present invention relates to use of the dispersible tablet of the present invention for manufacture of a medicament for the treatment or prevention of a proliferative disease, preferably a cancer.

In one embodiment, the present invention relates to dispersible tablets of the present invention for use in a method of treatment or prevention of a proliferative disease comprising administering to a patient in need thereof one or more dispersible tablets of the present invention that together comprise a therapeutically effective amount of Compound I or a pharmaceutically acceptable salt thereof.

In one embodiment, the present invention further relates to a medicament package comprising dispersible tablets according to the present invention and printed instructions directing one or more dispersible tablets of Compound I or a pharmaceutically acceptable salt thereof be administered orally.

### Detailed Description of the Invention

The present invention provides a dispersible tablet comprising (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of 5% to 50% in weight based on the total weight of the tablet, (b) sodium starch glycolate in an amount of 2% to 12% in weight based on the total weight of the tablet, (c) low-substituted hydroxypropyl cellulose in an amount of 1% to 10% in weight based on the total weight of the tablet, (d) sodium stearyl fumarate in an amount of 1% to 7% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

By "dispersible tablet" is meant an uncoated or film-coated tablet which disperses in aqueous medium, e.g., in water, soda or juice (e.g or vegetable juice), before administration.

Preferably, the dispersible tablet of the present invention comprises granulates having an inner phase and outer phase, wherein the inner phase comprises (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, sodium starch glycolate, low-substituted hydroxypropyl cellulose, and optionally any additional excipients, wherein the outer phase comprises sodium starch glycolate, sodium stearyl fumarate and optionally any additional excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

The present invention provides a dispersible tablet with high drug loading comprising Compound I or a pharmaceutically acceptable salt thereof as active ingredient. Compound I or a pharmaceutically acceptable salt thereof is present in an amount from 5% to 50%, e.g., from about 10% to 50% or 20% to 50% or 30% to 45%, preferably 35% to 45% in weight based on the total weight of the dispersible tablet. In particular, the amount of Compound I or a pharmaceutically acceptable salt thereof may range from 35 to 45%, e.g., about 36% to 41%, in weight based on the total weight of the dispersible tablet.

Certain terms used herein are described below. Unless defined otherwise, all technical and scientific terms used herein have the same meaning as is commonly understood by one of skill in the art to which this invention belongs. The following general definitions shall apply in this specification, unless otherwise specified:
The terms "comprising" and "including" are used herein in their open-ended and non-limiting sense unless otherwise noted.

The terms "a" and "an" and "the" and similar references in the context of describing the invention (especially in the context of the following claims) are to be construed to cover both the singular and the plural, unless otherwise indicated herein or clearly contradicted by context. Where the plural form is used for compounds, salts, and the like, this is taken to mean also a single compound, salt, or the like.

The term "pharmaceutically acceptable" is defined herein to refer to those compounds, materials, excipients, compositions and/or dosage forms, which are, within the scope of sound medical judgment, suitable for contact with the tissues a subject, e.g., a mammal or human, without excessive toxicity, irritation allergic response and other problem complications commensurate with a reasonable benefit / risk ratio.

The term "daily dosage" refers to the total dosage amount of the therapeutic agent administered to a specific patient in any single day or twenty-four hour period.

The phrase "effective amount" or "therapeutically effective amount" is used herein to mean an amount sufficient to reduce by at least about 15 percent, preferably by at least 50 percent, more preferably by at least 90 percent, and most preferably prevent, a clinically significant deficit in the activity, function and response of the subject in need thereof. Alternatively, an effective amount or therapeutically effective amount is an amount sufficient to provide an observable improvement over the baseline clinically observable signs and symptoms of a disease.

The term "pharmaceutical composition" is defined herein to refer to a mixture or solution containing at least one active ingredient to be administered to a subject, e.g., a mammal or human, in order to prevent or treat a particular disease or condition affecting the subject.

By "inner phase" is meant the granulate phase or core including the active ingredient (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant, at least one binder, and optionally one or more additional pharmaceutically acceptable excipients. It is also known as the internal phase.

By "outer phase" is meant at least one disintegrant, at least one lubricant, and optionally one or more additional pharmaceutically acceptable excipients which are added to the inner phase (granulates). It is also known as the external phase.

By "total weight of the dispersible tablet" is meant the weight of a tablet being the inner and outer phase.

The term "about" or "approximately" shall have the meaning of within 10%, more preferably within 5%, of a given value or range.

The term "subject" refers to an animal. Typically the animal is a mammal. A subject also refers to for example, primates (e.g., humans), cows, sheep, goats, horses, dogs, cats, rabbits, rats, mice, fish, birds and the like. In certain embodiments, the subject is a primate. In yet other embodiments, the subject is a human.

The present invention relates to a dispersible tablet comprising (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) sodium starch glycolate in an amount of 2% to 12% in weight based on the total weight of the tablet, (c) low-substituted hydroxypropyl cellulose in an amount of 1% to 10% in weight based on the total weight of the tablet, (d) sodium stearyl fumarate in an amount of 1% to 7% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

Preferably, the dispersible tablet of the present invention comprises granulates having an inner phase and outer phase, wherein the inner phase comprises *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, sodium starch glycolate, low-substituted hydroxypropyl cellulose, and optionally any additional excipients, wherein the outer phase comprises sodium starch glycolate, sodium stearyl fumarate and optionally any additional excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

(S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) ("Compound I") is a specific 2-carboxamide cycloamino urea derivative compound that potently and selectively targets the alpha (α)-isoform of class IA PI3K and having the following chemical structure: 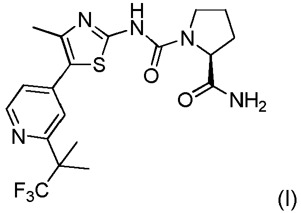 Compound I and its pharmaceutically acceptable salts are described in PCT Application No. WO2010/029082 and methods of its preparation have been described, for example, in Example 15 therein. Preferably, Compound I is in the free base form.

Compound I may be orally administered at an effective daily dose of about 1 to 6.5 mg/kg in human adults or children. Compound I may be orally administered to a 70 kg body weight human adult at a daily dosage of about 70 mg to 455 mg, e.g, about 200 to 400 mg, or about 240 mg to 400 mg, or about 300 mg to 400 mg, or about 350 mg to 400 mg, in a single dose or in divided doses up to four times a day. Preferably, Compound I is administered to a 70 kg body weight human adult at a daily dosage of about 350 mg to about 400 mg.

The present invention relates to a dispersible tablet with high drug loading comprising Compound I as active ingredient. Compound I is present in an amount from about 5% to 50%, e.g., from about 10% to 50% or about 20% to 50% or about 30% to 45%, preferably about 35% to 45% in weight based on the total weight of the dispersible tablet. In particular, the amount of Compound I may range from about 35 to 45%, e.g., about 36% to 41%, in weight based on the total weight of the dispersible tablet.

Suitable disintegrants for dispersible tablets include but are not limited to microcrystalline cellulose, sodium starch glycolate, low-substituted hydroxypropyl cellulose, sodium croscarmellose, calcium croscarmellose, cross-linked polyvinylpyrrolidone (e.g., as commercially available under the tradenames Crospovidone^{®} or Polyplasdone^{®} or Kollidone^{®}CL), cross-linked alginic acid, sodium alginate, potassium alginate, gellan gum, corn starch, pregelatinized starch, carboxymethylcellulose sodium, glycine and any combination thereof. Preferably, sodium starch glycolate or low -substituted hydroxypropyl cellulose is used. More preferably, both sodium starch glycolate and low-substituted hydroxypropyl cellulose are used.

The disintegrant may be sodium starch glycolate which is present in a total amount of 2% to 12%, preferably about 5% to 10%, in weight based on the total weight of the tablet. The disintegrant may also be a mixture of sodium starch glycolate which is present in a total amount of about 5% to 10% in weight based on the total weight of the tablet and low-substituted hydroxypropyl cellulose which is present in a total amount of about 2% to 5% in weight based on the total weight of the tablet.

In one embodiment, the disintegrant is sodium starch glycolate which is present in a total amount of about 2% to 12%, preferably about 5% to 10%, in weight based on the total weight of the tablet.

In one embodiment, the disintegrant consists of sodium starch glycolate which is present in a total amount of about 5% to 10% in weight based on the total weight of the tablet and low-substituted hydroxypropyl cellulose which is present in a total amount of about 2% to 5% in weight based on the total weight of the tablet.

According to the present invention, sodium starch glycolate is present in an amount of 2% to 12% in weight based on the total weight of the tablet.

Suitable binders for dispersible tablets include but are not limited to microcrystalline cellulose, low-substituted hydroxypropyl cellulose, hydroxypropylmethyl cellulose, hydroxymethylcellulose, hydroxyethyl cellulose, starch 1500, gum guar, gum xanthan, polyvinylpyrrolidone, sodium alginate and any combination thereof. Preferably, low-substituted hydroxypropyl cellulose is used.

A preferred binder is low-substituted hydroxypropyl cellulose. According to the present invention, low-substituted hydroxypropyl cellulose is present in a total amount of 1 to 10%, e.g., about 2 to 5%, e.g., about 1% to 3%, in weight based on the total weight of the tablet.

Suitable lubricants for dispersible tablets include but are not limited to sodium stearyl fumarate, magnesium stearate, stearic acid, hydrogenated castor oil, calcium stearate, aluminum stearate, PEG 4000-8000, talc, glyceryl monostearate, glyceryl dibehenate (e.g., commercially available by Gattefossé under trademark Compritol^{®} 888 ATO), glyceryl palmito-stearic ester (e.g., commercially available by Gattefossé under trademark Precerol^{®}), hydrogenated cotton seed oil, castor seed oil, and any combination thereof. Preferably, sodium stearyl fumarate is used.

According to the present invention, sodium stearyl fumarate is present in a total amount of 1% to 7%, e.g., about 3% to 6%, e.g., about 3% to 5%, in weight based on the total weight of the tablet.

The dispersible tablet of the present invention optionally may include one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose. Examples of pharmaceutically acceptable excipients that additionally may be used in the present invention include but are not limited to one or more diluents, with the proviso that the diluent is not calcium phosphate tribasic, calcium phosphate dibasic anhydrous, or calcium phosphate dibasic dihydrate; glidants; surfactants; taste masking agent, with the proviso that the taste masking agent is not sucralose, or any combination thereof.

Preferably, the dispersible tablet of the present invention further comprises an additional pharmaceutically acceptable excipient that is a diluent, with the proviso that that the diluent is not calcium phosphate tribasic, calcium phosphate dibasic anhydrous, or calcium phosphate dibasic dihydrate. Suitable diluents for the dispersible tablets of the present invention include but are not limited to mannitol (including the alpha, beta and delta polymorphs), sorbitol, maltodextrin, lactose (e.g., lactose monohydrate), microcrystalline cellulose, maltitol, xylitol, starch (e.g., corn, maize, or rice) or any combination thereof.

A preferred diluent is mannitol or microcrystalline cellulose, or a mixture of mannitol and microcrystalline cellulose. More preferably, the diluent is the delta polymorph of mannitol and/or microcrystalline cellulose.

The total amount of diluent may be in the range of about 20% to 70%, in particular, e.g., about 30% to 60%, e.g., about 35% to 55%, e.g., about 45% to 55%, e.g., about 45% to 50%, in weight based on the total weight of the tablet.

In one embodiment, the dispersible tablet of the present invention comprises an additional pharmaceutically acceptable excipient that is a diluent, with the proviso that that the diluent is not calcium phosphate tribasic, calcium phosphate dibasic anhydrous, or calcium phosphate dibasic dihydrate.

In one embodiment, the diluent is mannitol or microcrystalline cellulose. In one preferred embodiment, the diluent is mannitol and microcrystalline cellulose. In one preferred embodiment, the diluent is the delta polymorph of mannitol and/or microcrystalline cellulose.

In one embodiment, the tablet comprises an additional pharmaceutically acceptable excipient that is a diluent, wherein the diluent is present in a total amount in the range of about 30% to 60%, e.g., about 35% to 55%, e.g., about 45% to 55%, e.g., about 50% to 55%, in weight based on the total weight of the tablet, and wherein said diluent is mannitol and microcrystalline cellulose.

In one embodiment, the tablet comprises an additional pharmaceutically acceptable excipient that is a diluent, wherein the diluent is present in a total amount in the range of about 30% to 60%, e.g., about 35% to 55%, e.g., about 45% to 55%, e.g., about 50% to 55%, in weight based on the total weight of the tablet, and wherein said diluent is mannitol present in a total amount in the range of about 15% to 20% in weight based on the total weight of the tablet and microcrystalline cellulose present in a total amount in the range of about 25% to 35% in weight based on the total weight of the tablet.

The dispersible tablet of the present invention may comprise an additional pharmaceutically acceptable excipient that is a glidant. Suitable glidants for the dispersible tablets of the present invention include but are not limited to silica, colloidal silica (e.g., colloidal silica anhydrous), magnesium trisilicate, powdered cellulose, starch, talc and any combination thereof.

The total amount of glidant may be in the range of about 0.1% to 6%, in particular about 0.1% to 4%, e.g., about 0.1% to 2.5%, e.g., about 0.1 to 1.0%, in weight based on the total weight of the tablet.

In one embodiment, the dispersible tablet of the present invention comprises an additional pharmaceutically acceptable excipient that is a glidant.

In one embodiment, the tablet comprises an additional pharmaceutically acceptable excipient that is a glidant, wherein the total amount of glidant may be in the range of about 0.1% to 6%, in particular about 0.1% to 4%, e.g., about 0.1% to 2.5%, e.g., about 0.1 to 1.0%, in weight based on the total weight of the tablet.

The dispersible tablet of the present invention may comprise an additional pharmaceutically acceptable excipient that is a surfactant. Suitable surfactants for the dispersible tablets of the present invention include but are not limited to sodium lauryl sulfate, quaternary ammonium salts, polysorbates, sorbitan esters, poloxamer, vitamin E polyethylene glycol succinate, sucrose palmitate, docusate sodium, lecithin and any combination thereof.

The total amount of surfactant may be in the range of about 0.01% to 4%, in particular about 0.05% to 2.0%, e.g., about 0.05% to 1.0%, in weight based on the total weight of the tablet.

In one embodiment, the dispersible tablet of the present invention comprises an additional pharmaceutically acceptable excipient that is a surfactant.

In one embodiment, the dispersible tablet of the present invention comprises an additional pharmaceutically acceptable excipient that is a surfactant, wherein the total amount of surfactant may be in the range of about 0.01% to 4%, in particular about 0.05% to 2.0%, e.g., about 0.05% to 1.0%, in weight based on the total weight of the tablet.

The dispersible tablet of the present invention may comprise an additional pharmaceutically acceptable excipient that is a taste-masking agent, with the proviso that the additional pharmaceutical agent is not sucralose. A taste-masking agent can be a sweetener, a flavoring agent or a combination thereof.

A sweetener can be a sugar or a sugar substitute selected from lactose, mannitol, sucrose, glucose, thaumatin, neotame, tagatose, acesulfame potassium, saccharin sodium, aspartame, trehalose, saccharine or a combination thereof.

A flavoring agent is a substance capable of enhancing taste or aroma of a composition. Suitable flavoring agents can be selected from standard reference books, for example Fenaroli's Handbook of Flavor Ingredients, 3rd edition (1995). Suitable flavoring agents for the dispersible tablet of the present invention include but are not limited to (i.) natural flavors, which can be simulated with natural or synthetic agents or combinations thereof, such as almond, anise, apple, apricot, bergamot, blackberry, blackcurrant, blueberry, cacao, caramel, cherry, cinnamon, cranberry, eucalyptus, fig, ginger, grape, lemon, licorice, lime, malt, molasses, nutmeg, peach, pear, peppermint, pineapple, raspberry, rose, spearmint, strawberry, tangerine, tea, vanilla, wintergreen, etc., and (ii) synthetic flavors, such as tutti-frutti or bubblegum flavor. Flavoring agents can be used singly or in combinations of two or more.

One or more pharmaceutically acceptable excipients may be selected and used in the accordance with the present invention by one skilled in the art having regard to the particular desired properties of the dispersible tablet based on the present description and the experience and knowledge of one skilled in the art. In addition, the absolute amounts of each pharmaceutically acceptable excipient and the amounts relative to other excipients in the dispersible tablet of the present invention may be selected by one skilled in the art having regard to the particular desired properties of the dispersible tablet based on the present description and the experience and knowledge of one skilled in the art.

It will be appreciated that any given excipient may serve more than one function, e.g., as disintegrant, lubricant, binder, diluent, glidant and/or surfactant. For example, in a preferred embodiment, low-substituted hydroxypropyl cellulose is functioning as both a disintegrant and a binder.

The dispersible tablet of the present invention can be uncoated or film-coated. Suitable film coatings are known and commercially available or can be made according to known methods. Typically, film coating materials are hydrophilic polymers such as polyethylene glycol, polyvinylpyrrolidone, polyvinyl alcohol, hydroxypropylcellulose, hydroxymethylcellulose, and hydroxypropylmethylcellulose or the like. The film coating composition ingredients may include plasticizers in conventional amounts, as well as opacifiers and colorants. Typically, a film coating material is applied in such amount as to provide a film coating that ranges from about 1% to about 6% by weight of the total tablet. Dry mixtures such as Sepifilm or Opadry mixtures prepared by Colorcon Corp. may be used. These products are individually prepared dry pre-mixtures of film forming polymers, opacifiers, colorants and plasticizers which are further processed to aqueous film coating suspensions.

The film coating may be applied by conventional techniques in a suitable coating pan or fluidized bed apparatus using water and/or conventional organic solvents (e.g, methyl alcohol, ethyl alcohol, isopropyl alcohol), ketones (acetone), etc.

Preferably, the dispersible tablet of the present invention is uncoated.

The present invention relates to a dispersible tablet comprising (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of 5% to 50% in weight based on the total weight of the tablet, (b) sodium starch glycolate in an amount of 2% to 12% in weight based on the total weight of the tablet, (c) low-substituted hydroxypropyl cellulose in an amount of 1% to 10 % in weight based on the total weight of the tablet, and (d) sodium stearyl fumarate in an amount of 1% to 7 % in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

In a preferred embodiment, the present invention relates to a dispersible tablet comprising (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 35% to 45% in weight based on the total weight of the tablet, (b) sodium starch glycolate in an amount of about 5% to 10% in weight based on the total weight of the tablet, (c) low-substituted hydroxypropyl cellulose in an amount of about 1% to 3% in weight based on the total weight of the tablet, and (d) sodium stearyl fumarate in an amount of about 3% to 5% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

In a preferred embodiment, the present invention relates to a dispersible tablet comprising granulates having an inner phase and outer phase, wherein the inner phase comprises (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant sodium starch glycolate, at least one binder low-substituted hydroxypropyl cellulose, and optionally any additional excipients, wherein the outer phase comprises at least one disintegrant sodium starch glycolate, at least one lubricant sodium stearyl fumarate, and optionally any additional excipients, and wherein the dispersible tablet comprises (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of 5% to 50% in weight based on the total weight of the tablet, (b) sodium starch glycolate in an amount of 2% to 12% in weight based on the total weight of the tablet, (c) low-substituted hydroxypropyl cellulose in an amount of 1% to 10 % in weight based on the total weight of the tablet, and (d) sodium stearyl fumarate in an amount of 1% to 7 % in weight based on the total weight of the tablet, and optionally (e) one or more additional pharmaceutically acceptable excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

In a preferred embodiment, the present invention relates to a dispersible tablet comprising granulates having an inner phase and outer phase, wherein the inner phase comprises (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant sodium starch glycolate, at least one binder low-substituted hydroxypropyl cellulose, and optionally any additional excipients, wherein the outer phase comprises at least one disintegrant sodium starch glycolate, at least one lubricant sodium stearyl fumarate, and optionally any additional excipients, and wherein the dispersible tablet comprises (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 35% to 45% in weight based on the total weight of the tablet, (b) sodium starch glycolate in an amount of about 5% to 10% in weight based on the total weight of the tablet, (c) low-substituted hydroxypropyl cellulose in an amount of about 1% to 3% in weight based on the total weight of the tablet, and (d) sodium stearyl fumarate in an amount of about 3% to 5% in weight based on the total weight of the tablet, and optionally (e) one or more additional pharmaceutically acceptable excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

In accordance with the present invention, the dispersible tablets of the present invention show rapid disintegration in an aqueous medium such as water, soda or juice (e.g., fruit or vegetable juice). The rapid disintegration may be observed in standard tests known to one skilled in the art. The disintegration time is preferably measured according to the standard test for dispersible tablets described in European Pharmacopoeia Eighth edition, Supplement 8.2, page 811 (January 2014) in combination with European Pharmacopoeia, Eighth edition, Supplement 8.2, page 285-287, Section 2.9.1 (January 2014). By "disintegration time" is meant the time that needs the dispersible tablet to disintegrate in water at room temperature in a disintegration time device. This test from the European Pharmacopoeia (January 2014) examines the disintegration time of tablets in water at 15 to 25 °C.

The rate and completion of disintegration may be observed visually. Disintegration is considered to be achieved when no residue remains on the screen of the test apparatus, or if there is residue remaining on the screen of the test apparatus, it consists of a soft mass having no palpably firm core, or only fragments of the insoluble coating remain on the screen.

The tablets of the present invention preferably have a disintegration time of 5 minutes or less, preferably 3 minutes or less, when measured according to the above test in water at a temperature of 15 to 25 °C. More preferably, the disintegration time is 90 seconds or less.

Preferably, the dispersible tablets of the present invention produce a smooth dispersion which passes through a sieve screen with a nominal mesh aperture of 710 µm after said tablets are completely dispersed in aqueous medium such as water, soda or juice (e.g., fruit or vegetable juice). This fineness of dispersion may be measured according to the standard test for dispersible tablets described in European Pharmacopoeia Eighth edition, Supplement 8.2, page 811 (January 2014).

In accordance with the present invention, the dispersible tablets of the present invention are dispersed in ingestible liquid, including but not limited to water, soda, and juice (e.g., fruit juice or vegetable juice), prior to administration to the subject. Preferably, the ingestible liquid is water. The resulting dispersed tablet may be administered to a subject or patient by oral ingestion or by administration via a feeding tube, preferably a gastronomy tube (G-tube) or PEG (percutaneous endoscopic gastrostomy) tube.

In one aspect, the dispersible tablets of the present invention is administered to a patient, such as a child or an adult suffering from a proliferative disease (e.g., a cancer), by (i) contacting said tablet with an ingestible liquid, (ii) allowing the tablet to disperse in the ingestible liquid to form a dispersed mixture, e.g., in 5 minutes or less, preferably 3 minutes or less, and (iii) ingesting the dispersed mixture.

In one embodiment, the present invention relates to a method of administering the dispersible tablet of the present invention to a patient in need thereof which comprises (i) contacting tablet with an ingestible liquid, (ii) dispersing the tablet in the ingestible liquid to form a dispersed mixture, e.g., in 5 minutes or less, preferably 3 minutes or less, and (iii) ingesting the dispersed mixture.

In one embodiment, the present invention relates to a method of administering the dispersible tablet of the present invention to a patient in need thereof which comprises instructing the subject in need thereof to (i) contact tablet with an ingestible liquid, (ii) disperse the tablet in the ingestible liquid to form a dispersed mixture, e.g., in 5 minutes or less, preferably 3 minutes or less, and (iii) ingest the dispersed mixture.

In another aspect, the dispersible tablets of the present invention may be administered to a patient in need thereof by (i) contacting said tablet with an ingestible liquid (ii) allowing the tablet to disperse in the ingestible liquid to form a dispersed mixture, e.g., in 5 minutes or less, preferably 3 minutes or less, and (iii) administering said dispersed mixture said patient using or via a feeding tube, preferably a gastronomy tube (G-tube) or PEG (percutaneous endoscopic gastrostomy) tube.

In one embodiment, the present invention relates to a method of administering the dispersible tablet of the present invention to a patient in need which comprises (i) contacting said tablet with an ingestible liquid (ii) dispersing the tablet in the ingestible liquid to form a dispersed mixture, e.g., in 5 minutes or less, preferably 3 minutes or less, and (iii) administering said dispersed mixture said patient using or via a feeding tube, preferably a gastronomy tube (G-tube) or PEG tube.

The dispersible tablets of the present invention may be dispersed in, e.g., 20 to 50 ml water with stirring.

In accordance with the present invention, it has been surprisingly found that these dispersible tablets having high drug loading (e.g., from about 5% to 50%, preferably about 35% to 45%, weight based on the total weight of the tablet) of Compound I and having dispersibility in aqueous medium, such as water, soda or juice (e.g., fruit or vegetable juice), in 5 minutes or less, preferably 3 minutes or less, may be obtained by wet granulation followed by compression methods.

Typically, wet granulation is not preferred for preparing dispersible tablets. Wet-granulation increases the cohesion of the active ingredient particles and increases the disintegration time of the final tablet which is not in accordance with patient compliance or the *European Pharmacopoeia* which requests a disintegration time of 3 minutes or less for a dispersible tablet. It has been surprisingly found that the specific use of Compound I with a disintegrant in a total amount of about 1% to 25% in weight based on the total weight of the tablet, a binder in a total amount of about 1% to 20% in weight based on the total weight of the tablet, and a lubricant in a total amount of about 1% to 15% in weight based on the total weight of the tablet produces a stable composition with high drug loading (e.g., from about 5% to 50%, preferably about 35% to 45%, weight based on the total weight of the tablet) and which disperses in aqueous medium in 5 minutes or less, preferably 3 minutes or less. Further, it has been surprisingly found that Compound I must be prepared as a dispersible tablet that does not include calcium phosphate tribasic, calcium phosphate dibasic anhydrous, calcium phosphate dibasic dihydrate or sucralose to achieve the foregoing physical and chemical properties and suitable stability.

The present invention relates to a process for producing a dispersible tablet of the present invention, comprising
(a) forming a granulate having an inner phase and an outer phase by
   (i.) wet granulating an inner phase comprising (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, sodium starch glycolate in a total amount of 1% to 7% in weight based on the total weight of the tablet, low substituted hydroxypropyl cellulose in a total amount of 1% to 10% in weight based on the total weight of the tablet, and at least one diluent;
   (ii.) adding sodium starch glycolate in a total amount of 1% to 5% in weight based on the total weight of the tablet and at least one diluent to the inner phase formed in step (a)(i.) and mixing; and
   (iii.) adding sodium stearyl fumarate in a total amount of 1% to 7% in weight based on the total weight of the tablet to the mixture formed in step (a)(ii.) and mixing; and
(b) forming the dispersible tablet by compressing the mixture obtained in step (a)(iii.), with the proviso that the diluent according to step (a)(i.) or (a)(ii.) is not calcium phosphate tribasic, calcium phosphate dibasic anhydrous, or calcium phosphate dibasic dihydrate.

Preferably, the diluent in step (a)(i.) is mannitol and microcrystalline cellulose and the diluent in step (a)(ii.) is microcrystalline cellulose.

In one embodiment, the diluent in step (a)(i.) is mannitol which is present in a total amount of about 15% to 20% in weight based on the total weight of the tablet and microcrystalline cellulose which is present in an amount of about 15% to 20% in weight based on the total weight of the tablet, and the diluent in step (a)(ii.) is microcrystalline cellulose which is present in an amount of about 10% to 15% in weight based on the total weight of the tablet.

In one embodiment, the process further comprises the step of applying a film-coat to the tablet.

Procedures which may be used may be conventional or known in the art or based on such procedures, e.g., those described in L. Lachman et al. The Theory and Practice of Industrial Pharmacy, 3rd Ed., 1986; H. Sucker et al., Pharmazeutische Technologie, Thieme, 1991, Hagers, Handbuch der Pharmazeutischen Praxis, 4th Ed. (Springer Verlag, 1971) and Remington's pharmaceutical Sciences, 13th Ed. (Mack Publ., Co., 1970) or later editions.

In a further embodiment, the present invention relates to a process for producing a dispersible tablet of the present invention, comprising
(a) forming a granulate having an inner phase and an outer phase by
   (i.) mixing Compound I or a pharmaceutically acceptable salt thereof in an amount of about 5% to 50% in weight based on the total weight of the tablet, sodium starch glycolate in a total amount of about 1% to 7% in weight based on the total weight of the tablet, and at least one diluent in a high-shear granulator,
   (ii.) adding a solution comprising low-substituted hydroxypropyl cellulose to the mixture of step (a)(i.), subjecting the mixture to wetting and kneading in a high-shear granulator, wherein low-substituted hydroxypropyl cellulose is added to the mixture of step (a)(i.) in a total amount of about 1% to 10% in weight based on the total weight of the tablet
   (iii.) wet sieving said mixture of step (a)(ii.) in a screening mill,
   (iv.) drying the resulting mixture of step (a)(iii) in a fluidized bed dryer,
   (v.) screening the resulting dried mixture of step (a)(iv.) in a screening mill with oscillating bar,
   (vi.) adding sieved excipients comprising sodium starch glycolate in an amount of about 1% to 5% in weight based on the total weight of the tablet, sodium stearyl fumarate in an amount of about 1% to 7% in weight based on the total weight of the tablet, and at least one diluent to the resulting mixture of step (v.) and mix in a diffusion mixture (tumble), and
(b) tableting the mixture from step (vi.) by compression using a conventional tableting press, preferably a rotary tableting press,
with the proviso that said diluent according to steps (a)(i.), (a)(vi.) is not calcium phosphate tribasic, calcium phosphate dibasic anhydrous, calcium phosphate dibasic dihydrate or sucralose.

In one embodiment, the step (a)(i.) comprises a diluent consisting of mannitol which is present in a total amount of about 15% to 20% in weight based on the total weight of the tablet and microcrystalline cellulose which is present in an amount of about 15% to 20% in weight based on the total weight of the tablet, and step (a)(i.) comprises the diluent microcrystalline cellulose which is present in an amount of about 10% to 15% in weight based on the total weight of the tablet.

In one aspect, the present invention relates to a dispersible tablet made or obtained by the process described above. Preferably, the dispersible tablet is uncoated.

The physical and chemical stability may be tested in a conventional manner, e.g., the dispersible tablets may be tested as such by measurement of dissolution, friability, disintegration time, assay for Compound I degradation products, appearance and/or microscopy, e.g., after storage at room temperature, i.e., 25°C, and/or storage at 40°C.

The dispersible tablets may vary in shape and be, for example, round, oval, oblong, cylindrical or any other suitable shape. In a preferred embodiment of the present invention, the dispersible tablets obtained by the process described above are of round shape. The edges of the dispersible tablets may be beveled or rounded. More preferably, the dispersible tablets are of round shape or oval shape with beveled edges. The dispersible tablets may be scored, embossed or engraved.

The dispersible tablets of the present invention are preferably of round shape or oval shape, embossed, and with beveled edges.

The dispersible tablets of the present invention may be colored and/or marked as to impart an individual appearance and to make them instantly recognizable. The use of dyes can serve to enhance the appearance as well as to identify the dispersible tablets. Dyes suitable for use in pharmaceutical compositions or dosage forms typically include carotinoids, iron oxides or chlorophyll. The dispersible tablets of the present invention may be marked using an imprint code.

The hardness, or resistance to crushing, of tablets according to the present invention may be determined by standard tests. A device such as a Kraemer^{®} 3S tablet testing device may be used. This test determines the resistance to crushing of tablets, measured by the force needed to disrupt them by crushing.

The hardness of the tablets of the present invention varies according to the weight and diameter of the tablets and the compression force. For a tablet comprising about 50 mg of Compound I as active ingredient, having a diameter of about 7 mm, the hardness is preferably from about 25 N to 75N, preferably about 35 N to 65 N, most preferably about 50N and may be achieved by applying a compression force of about 5 to 11 kN. For a tablet comprising about 200 mg of Compound I as active ingredient, having a diameter of about 6.3 mm, the hardness is preferably from about 120N to 180N, preferably from about 130N to 170N, most preferably about 150N and may be achieved by applying a compression force of about 12 to 20 kN. For other tablet weights and diameters, the preferred hardness varies.

Thus, the advantageous properties of the dispersible tablets of the present invention may be demonstrated by the hardness and disintegration times of said tablets. Accordingly, in a preferred embodiment, the present invention comprises a dispersible tablet as defined above comprising 50 mg of Compound I or a pharmaceutically acceptable salt thereof, when compressed using a force of 5 to 11 kN with a 7 mm diameter die and standard round punches, has a hardness of 25 to 75 N and a disintegration time of 3 minutes or less. Accordingly, in a preferred embodiment, the present invention comprises a dispersible tablet as defined above comprising 200 mg of Compound I or a pharmaceutically acceptable salt thereof, when compressed using a force of 12 to 20 kN with a 16.0 x 6.3 mm die and standard ovaloid punches, has a hardness of 120 to 180 N and a disintegration time of 3 minutes or less.

The above statements of the present invention define the dispersible tablet in terms of the properties of a particular tablet made from the described pharmaceutical composition. However, it is clear that the invention is in no way thereby limited to tablets having such a weight, diameter, or hardness, or only to a production process involving the use of such a compression force. As discussed definition is rather given in order to clarify that the advantageous intrinsic properties of the formed tablet are including a rapid disintegration time in combination with a good degree of hardness.

The dispersible tablets of the present invention are useful for the treatment or prevention of a proliferative disorder, preferably a cancer.

The dispersible tablets of the present invention are particularly useful for the treatment or prevention of cancers including, for example, sarcoma, cancers of the lung, bronchus, prostate, breast (including sporadic breast cancers and sufferers of Cowden disease), pancreas, gastrointestine, colon, rectum, colon carcinoma, colorectal adenoma, thyroid, liver, intrahepatic bile duct, hepatocellular, adrenal gland, stomach, gastric, glioma, glioblastoma, endometrial, melanoma, kidney, renal pelvis, urinary bladder, uterine corpus, uterine cervix, vagina, ovary, multiple myeloma, esophagus, a leukemia, acute myelogenous leukemia, chronic myelogenous leukemia, lymphocytic leukemia, myeloid leukemia, brain, oral cavity and pharynx, larynx, small intestine, non-Hodgkin lymphoma, melanoma, villous colon adenoma, a neoplasia, a neoplasia of epithelial character, lymphomas, a mammary carcinoma, basal cell carcinoma, squamous cell carcinoma, actinic keratosis, head and neck, polycythemia vera, essential thrombocythemia, myelofibrosis with myeloid metaplasia, and Waldenstroem disease. Preferably, the dispersible tablets of the present invention are used for treatment of a proliferative disease.

Proliferative diseases mediated by the alpha-subunit of PI3K may include those showing overexpression or amplification of PI3K alpha, somatic mutation of PIK3CA or germline mutations or somatic mutation of PTEN or mutations and translocation of p85α that serve to up-regulate the p85-p110 complex. In a preferred embodiment, the cancer is a tumor and/or cancerous growth mediated by the alpha isoform of PI3K.

In one embodiment, the proliferative disease is a cancer selected from a cancer of the lung, bronchus, prostate, breast (including sporadic breast cancers and sufferers of Cowden disease), colon, rectum, colon carcinoma, colorectal adenoma, pancreas, gastrointestine, hepatocellular, stomach, gastric, ovary, squamous cell carcinoma, and head and neck.

In a preferred embodiment, the proliferative disease is a head and neck cancer.

In a preferred embodiment, the proliferative disease is breast cancer.

In a preferred embodiment, the dispersible tablets of the present invention are used for the treatment of a proliferative disorder, preferably a cancer.

In a further embodiment, the present invention relates to use of the dispersible tablet of the present invention for the treatment or prevention of a proliferative disease, preferably a cancer.

In a further embodiment, the present invention relates to dispersible tablets of the present invention for use in a method of treatment or prevention of a proliferative disease comprising administering to a patient in need thereof one or more dispersible tablets of the present invention that together comprise a therapeutically effective amount of Compound I or a pharmaceutically acceptable salt thereof. Preferably, a subject or patient in need of said dispersible tablets of the present inventions are those subjects or patients suffering from a proliferative disorder, preferably a cancer.

The activity and characteristics of the dispersible tablets of the present invention may be indicated in standard clinical trials and/or animal trials.

Depending on age, patient condition, mode of administration, and type of disease, Compound I is orally administered at an effective daily dosage of about 1 to 6.5 mg/kg in adults or children. In a 70 kg body weight adult patient, Compound I is orally administered at a daily dosage of about 70 mg to 455 mg. It will be understood that the specific dose level for any particular patient will depend on a variety of factors including the age, body weight, general health, drug combination with one or more active drugs, type and severity of the disease. With the teachings of the present invention, one skilled in the art would have the experience and skill to select the proper dose level treatment of a specific patient.

In one aspect, the present invention further relates to a medicament package comprising dispersible tablets according to the present invention and printed instructions directing one or more dispersible tablets of Compound I or a pharmaceutically acceptable salt thereof be administered orally.

The following Example illustrates the invention described above, but is not, however, intended to limit the scope of the invention in any way. Other test models known as such to the person skilled in the pertinent art can also determine the beneficial effects of the claimed invention.

### Example 1: Dispersible tablet formulation (50 mg dispersible tablets with a disintegration time below 3 minutes)

| | Component | % per tablet | Amount per tablet (mg) |
|---|---|---|---|
| Internal Phase | Compound I | 39.4 | 50.00 |
| | Microcrystalline cellulose (Avicel PH101) | 18.1 | 23.00 |
| | Mannitol (Mannitol delta) | 18.1 | 23.00 |
| | Sodium starch glycolate (Type A) | 2.4 | 3.00 |
| | Low-substituted hydroxypropyl cellulose | 2.4 | 3.00 |
| | | | |
| External Phase | Microcrystalline cellulose (MCC PH102) | 12.5 | 15.86 |
| | Sodium stearyl fumarate | 4.0 | 5.08 |
| | Sodium starch glycolate (Type A) | 3.2 | 4.06 |
| | Total Core weight (mg) | | 127.00 |

The tablet is prepared by forming a granulate having an inner phase and an outer phase by (i.) wet granulating an inner phase comprising 50.00 mg of Compound I, 23.00 mg of microcrystalline cellulose (Avicel PH101), 23.00 mg of mannitol, 3.0 mg of sodium starch glycolate, and 3.00 mg of low-substituted hydroxypropyl cellulose, and then (ii) forming the outer phase by blending the resulting mixture with sieved 4.06 mg of sodium starch glycolate and 15.86 mg of microcrystalline cellulose (Avicel PH102) and then by blending the resulting mixture with sieved 5.08 mg sodium stearyl fumarate.

The tablet is obtained by tableting the mixture obtained in step (ii) above. The composition obtained in step (ii) above is compressed with a Fette^{®} 102i rotary tablet press using a compression force of about 5 to 11 kN (preferably about 8 kN) with a 7 mm diameter die and standard round punches.

The resulting tablet has the following properties:

| Test | Release specification |
|---|---|
| Tablet appearance | 7 mm diameter, round, flat, beveled edge with engraving |
| Tablet appearance | White |
| Hardness (20 units) | Target: 50 N |
| | Range of Individual: 25 to 75 N |
| | Range of mean: 35 to 65 N |
| Friability (after 100 rotations) | Max 0.8% (0 unit broken) |
| Disintegration time | All tested units less than or equal to 3 minutes |
| | Method: without discs, purified water at room temperature (between 15 and 25°C) |
| Average mass | 121.9 - 132.1 mg |
| Fineness of dispersion | Smooth dispersion which passes through a sieve screen with a nominal mesh aperture of 710 µm |

### Example 2: Dispersible tablet formulation (200 mg dispersible tablets with a disintegration time below 3 minutes)

| | Component | % per tablet | Amount per tablet (mg) |
|---|---|---|---|
| Internal Phase | Compound I | 39.4 | 200.00 |
| | Microcrystalline cellulose (Avicel PH101) | 18.1 | 92.00 |
| | Mannitol (Mannitol delta) | 18.1 | 92.00 |
| | Sodium starch glycolate (Type A) | 2.4 | 12.00 |
| | Low-substituted hydroxypropyl cellulose | 2.4 | 12.00 |
| | | | |
| External Phase | Microcrystalline cellulose (MCC PH102) | 12.5 | 63.44 |
| | Sodium stearyl fumarate | 4.0 | 20.32 |
| | Sodium starch glycolate (Type A) | 3.2 | 16.24 |
| | Total Core weight (mg) | | 508.00 |

The tablet is prepared by forming a granulate having an inner phase and an outer phase by (i.) wet granulating an inner phase comprising 200.00 mg of Compound I, 92.00 mg of microcrystalline cellulose (Avicel PH101), 92.00 mg of mannitol, 12.00 mg of sodium starch glycolate, and 12.00 mg of low-substituted hydroxypropyl cellulose, and then (ii) forming the outer phase by blending the resulting mixture with sieved 16.24 mg of sodium starch glycolate and 63.44 mg of microcrystalline cellulose (Avicel PH102) and then by blending the resulting mixture with sieved 20.32 mg sodium stearyl fumarate.

The tablet is obtained by tableting the mixture obtained in step (ii) above. The composition obtained in step (ii) above is compressed with a Fette^{®} 102i rotary tablet press using a compression force of about 12 to 20 kN (preferably about 16 kN) with 16.0 x 6.3 mm die and standard ovaloid punches.

The resulting tablet has the following properties:

| Test | Release specification |
|---|---|
| Tablet appearance | 16.0 x 6.3 mm ovaloid flat, beveled edge with engraving |
| Tablet appearance | White |
| Hardness (20 units) | Target: 150 N |
| | Range of Individual: 120 to 180 N |
| | Range of mean: 130 to 170 N |
| Friability (after 100 rotations) | Max 0.8% (0 unit broken) |
| Disintegration time | All tested units less than or equal to 3 minutes |
| | Method: without discs, purified water at room temperature (between 15 and 25°C) |
| Average mass | 492.8 - 523.2 mg |
| Fineness of dispersion | Smooth dispersion which passes through a sieve screen with a nominal mesh aperture of 710 µm |

### Example 3: Examples of Disintegration Times

The following table provides examples of disintegration times in minutes of tablets formulated according to Example 1 or Example 2:

| Test No. | Tablets Tested | Batch size that tested dispersible tablets were sampled from | Disintegration time |
|---|---|---|---|
| 1 | 50 mg dispersible tablet according to Example 1 | Approximately 1-2 kg | 1 minute |
| 2 | 50 mg dispersible tablet according to Example 1 | Approximately 20 kg | 1 minute |
| 3 | 200 mg dispersible tablets according to Example 2 | Approximately 1-2 kg | 1 minute |
| 4 | 200 mg dispersible tablets according to Example 2 | Approximately 74.3 kg | 2 minutes |

In the above tests, the specified tablets formulated according to Example 1 or Example 2 are evaluated to determine whether the tablets disintegrate within less than five minutes when placed in a liquid medium. The disintegration time of the tablets are evaluated according to the procedure set forth in European Pharmacopoeia, Eighth edition, Supplement 8.2, page 285-287, Section 2.9.1 (January 2014). No disks were used in these experiments.

Complete disintegration is defined as that state in which any residue of the tablet, except fragments of insoluble coating, remaining on the screen of the test apparatus is a soft mass having no palpably firm core.

A basket-rack assembly is used for these tests. The basket-rack assembly consists of 6 open-ended transparent tubes (each approximately 77.5 ± 2.5 mm long and having a diameter of approximately 21.85 ± 1.15 mm and a wall approximately 1.9 ± 0.9 mm thick) held by 2 plates having 6 holds equidistant from the center of the plate and equally spaced from another. Attached to the under surface of the lower plate is a woven stainless steel wire cloth, which has a plain square weave with 2.0 ± 0.2 mm mesh apertures and wit a wire diameter of 0.615 ± 0.045 mm. The parts of the apparatus are assembled and rigidly held by means of 3 bolts passing through the 2 plates. The basket-rack assembly is suspended from the raising and lowering device using a point on its axis. It is understood that the design of the basket-rack assembly may be varied provided that the specifications for the glass tubes and the screen mesh size are maintained.

In the disintegration test, one tablet is placed in each of the 6 tubes of the basket-rack assembly. The apparatus is operated using purified water at room temperature (approximately 15 to 25°C), as the immersion fluid. The tablets are evaluated for level and rate of disintegration by lifting the basket from the liquid. The results provided above reflect the maximum disintegration time from one tablet out of the six tablets in the disintegration test.

## Claims

1. A dispersible tablet comprising (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of 5% to 50% in weight based on the total weight of the tablet, (b) sodium starch glycolate in an amount of 2% to 12% in weight based on the total weight of the tablet, (c) low-substituted hydroxypropyl cellulose in an amount of 1% to 10% in weight based on the total weight of the tablet, (d) sodium stearyl fumarate in an amount of 1% to 7% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

2. A dispersible tablet according to claim 1 comprising granulates having an inner phase and outer phase, wherein the inner phase comprises (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, sodium starch glycolate, low-substituted hydroxypropyl cellulose, and optionally any additional pharmaceutically acceptable excipients, wherein the outer phase comprises sodium starch glycolate, sodium stearyl fumarate and optionally any additional pharmaceutically acceptable excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

3. A dispersible tablet according to any one of the preceding claims, wherein the sodium starch glycolate is present in a total amount from 5% to 12%, e.g., 5 to 10% in weight based on the total weight of the tablet.

4. A dispersible tablet according to any one of the preceding claims, wherein the low-substituted hydroxypropyl cellulose is present in a total amount from 2% to 5%, e.g., 1% to 3%, in weight based on the total weight of the tablet.

5. A dispersible tablet according to any one of the preceding claims, wherein the sodium stearyl fumarate is present in a total amount from 3% to 6%, e.g., 3% to 5%, in weight based on the total weight of the tablet.

6. A dispersible tablet according to any one of the preceding claims, wherein the dispersible tablet further comprises an additional pharmaceutically acceptable excipient that is a diluent, with the proviso that that the diluent is not calcium phosphate tribasic, calcium phosphate dibasic anhydrous, or calcium phosphate dibasic dihydrate.

7. A dispersible tablet according to claim 6, wherein the diluent is selected from mannitol (including the alpha, beta and delta polymorphs), sorbitol, maltodextrin, lactose (e.g., lactose monohydrate), microcrystalline cellulose, maltitol, xylitol, starch (e.g., corn, maize, or rice) or any combination thereof.

8. A dispersible tablet according to claim 7, wherein the total amount of diluent is in the range of 20% to 70%, e.g., 30% to 60%, e.g., 35% to 55%, e.g., 45% to 55%, e.g., 45% to 50%, in weight based on the total weight of the tablet.

9. A dispersible tablet according to any one of the preceding claims, wherein the tablet has a disintegration time of 5 minutes or less, preferably 3 minutes or less, when measured in water at a temperature of 15 to 25 °C according to the standard test for dispersible tablets described in European Pharmacopoeia Eighth edition, Supplement 8.2, page 811, January 2014, in combination with European Pharmacopoeia, Eighth edition, Supplement 8.2, page 285-287, Section 2.9.1, January 2014.

10. A dispersible tablet according to any one of the preceding claims for use in the treatment or prevention of a proliferative disease, preferably a cancer.

11. A process for producing a dispersible tablet of claim 1, comprising
(a) forming a granulate having an inner phase and an outer phase by
(i.) wet granulating an inner phase comprising (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of 5% to 50% in weight based on the total weight of the tablet, sodium starch glycolate in a total amount of 1% to 7% in weight based on the total weight of the tablet, low-substituted hydroxypropyl cellulose in a total amount of 1% to 10% in weight based on the total weight of the tablet, and at least one diluent;
(ii.) adding sodium starch glycolate in a total amount of 1% to 5% in weight based on the total weight of the tablet, and at least one diluent to the inner phase formed in step (a)(i.) and mixing; and
(iii.) adding sodium stearyl fumarate in a total amount of 1% to 7% in weight based on the total weight of the tablet to the mixture formed in step (a)(ii.) and mixing; and
(b) forming the dispersible tablet by compressing the mixture obtained in step (a)(iii.),
with the proviso that the diluent according to step (a)(i.) or (a)(ii.) is not calcium phosphate tribasic, calcium phosphate dibasic anhydrous or calcium phosphate dibasic dihydrate.

## Patentansprüche

1. Dispergierbare Tablette, umfassend (a) (S)-Pyrrolidin-l,2-dicarbonsäure-2-amid-l-({4-methyl-5-[2-(2,2,2-trifluor-1,1-dimethylethyl)pyridin-4-yl]thiazol-2-yl}amid) oder ein pharmazeutisch unbedenkliches Salz davon in einer Menge von 5 bis 50 Gew.-%, bezogen auf das Gesamtgewicht der Tablette, (b) Natriumstärkeglykolat in einer Menge von 2 bis 12 Gew.-%, bezogen auf das Gesamtgewicht der Tablette, (c) niedrigsubstituierte Hydroxypropylcellulose in einer Menge von 1 bis 10 Gew.-%, bezogen auf das Gesamtgewicht der Tablette, (d) Natriumstearylfumarat in einer Menge von 1 bis 7 Gew.-%, bezogen auf das Gesamtgewicht der Tablette, und gegebenenfalls (e) einen oder mehrere zusätzliche pharmazeutisch unbedenkliche Hilfsstoffe, mit der Maßgabe, dass es sich bei dem zusätzlichen pharmazeutisch unbedenklichen Hilfsstoff nicht um Calciumphosphat, wasserfreies Calciumhydrogenphosphat, Calciumhydrogenphosphatdihydrat oder Sucralose handelt.

2. Dispergierbare Tablette nach Anspruch 1, umfassend Granulate mit einer inneren Phase und einer äußeren Phase, wobei die innere Phase (S)-Pyrrolidin-1,2-dicarbonsäure-2-amid-1-({4-methyl-5-[2-(2,2,2-trifluor-1,1-dimethylethyl)pyridin-4-yl]thiazol-2-yl}amid) oder ein pharmazeutisch unbedenkliches Salz davon, Natriumstärkeglykolat, niedrigsubstituierte Hydroxypropylcellulose und gegebenenfalls zusätzliche pharmazeutisch unbedenkliche Hilfsstoffe umfasst, wobei die äußere Phase Natriumstärkeglykolat, Natriumstearylfumarat und gegebenenfalls zusätzliche pharmazeutisch unbedenkliche Hilfsstoffe umfasst, mit der Maßgabe, dass es sich bei dem zusätzlichen pharmazeutisch unbedenklichen Hilfsstoff in der inneren Phase oder äußeren Phase nicht um Calciumphosphat, wasserfreies Calciumhydrogenphosphat, Calciumhydrogenphosphatdihydrat oder Sucralose handelt.

3. Dispergierbare Tablette nach einem der vorhergehenden Ansprüche, wobei das Natriumstärkeglykolat in einer Gesamtmenge von 5 bis 12 Gew.-%, z. B. 5 bis 10 Gew.-%, bezogen auf das Gesamtgewicht der Tablette, vorliegt.

4. Dispergierbare Tablette nach einem der vorhergehenden Ansprüche, wobei die niedrigsubstituierte Hydroxypropylcellulose in einer Gesamtmenge von 2 bis 5 Gew.-%, z. B. 1 bis 3 Gew.-%, bezogen auf das Gesamtgewicht der Tablette, vorliegt.

5. Dispergierbare Tablette nach einem der vorhergehenden Ansprüche, wobei das Natriumstearylfumarat in einer Gesamtmenge von 3 bis 6 Gew.-%, z. B. 3 bis 5 Gew.-%, bezogen auf das Gesamtgewicht der Tablette, vorliegt.

6. Dispergierbare Tablette nach einem der vorhergehenden Ansprüche, wobei die dispergierbare Tablette ferner einen zusätzlichen pharmazeutisch unbedenklichen Hilfsstoff, bei dem es sich um ein Verdünnungsmittel handelt, umfasst, mit der Maßgabe, dass es sich bei dem Verdünnungsmittel nicht um Calciumphosphat, wasserfreies Calciumhydrogenphosphat oder Calciumhydrogenphosphatdihydrat handelt.

7. Dispergierbare Tablette nach Anspruch 6, wobei das Verdünnungsmittel aus Mannitol (einschließlich der alpha-, beta- und delta-Polymorphe), Sorbitol, Maltodextrin, Lactose (z. B. Lactosemonohydrat), mikrokristalline Cellulose, Maltitol, Xylitol, Stärke (z. B. Mais oder Reis) oder einer beliebigen Kombination davon ausgewählt ist.

8. Dispergierbare Tablette nach Anspruch 7, wobei die Gesamtmenge an Verdünnungsmittel im Bereich von 20 bis 70 Gew.-%, z. B. 30 bis 60 Gew.-%, z. B. 35 bis 55 Gew.-%, z. B. 45 bis 55 Gew.-%, z. B. 45 bis 50 Gew.-%, bezogen auf das Gesamtgewicht der Tablette, liegt.

9. Dispergierbare Tablette nach einem der vorhergehenden Ansprüche, wobei die Tablette bei Messung in Wasser bei 15 bis 25 °C gemäß dem in der Europäischen Pharmakopöe, achte Auflage, Ergänzungsband 8.2, Seite 811, Januar 2014, beschriebenen Standardtest für dispergierbare Tabletten in Kombination mit der Europäischen Pharmakopöe, achte Auflage, Ergänzungsband 8.2, Seite 285-287, Abschnitt 2.9.1, Januar 2014, eine Zerfallszeit von 5 Minuten oder weniger, vorzugsweise 3 Minuten oder weniger, aufweist.

10. Dispergierbare Tablette nach einem der vorhergehenden Ansprüche zur Verwendung bei der Behandlung oder Prävention einer proliferativen Erkrankung, vorzugsweise einer Krebserkrankung.

11. Verfahren zur Herstellung einer dispergierbaren Tablette nach Anspruch 1, das Folgendes umfasst:
(a) Bilden eines Granulats mit einer inneren Phase und einer äußeren Phase durch
(i.) Feuchtgranulieren einer inneren Phase, umfassend (S)-Pyrrolidin-1,2-dicarbonsäure-2-amid-1-({4-methyl-5-[2-(2,2,2-trifluor-1,1-dimethylethyl)pyridin-4-yl]thiazol-2-yl}amid) oder ein pharmazeutisch unbedenkliches Salz davon in einer Menge von 5 bis 50 Gew.-%, bezogen auf das Gesamtgewicht der Tablette, Natriumstärkeglykolat in einer Gesamtmenge von 1 bis 7 Gew.-%, bezogen auf das Gesamtgewicht der Tablette, niedrigsubstituierte Hydroxypropylcellulose in einer Gesamtmenge von 1 bis 10 Gew.-%, bezogen auf das Gesamtgewicht der Tablette, und mindestens ein Verdünnungsmittel;
(ii.) Zugeben von Natriumstärkeglykolat in einer Gesamtmenge von 1 bis 5 Gew.-%, bezogen auf das Gesamtgewicht der Tablette, und mindestens eines Verdünnungsmittels zu der in Schritt (a) (i.) gebildeten inneren Phase und Mischen; und
(iii.) Zugeben von Natriumstearylfumarat in einer Gesamtmenge von 1 bis 7 Gew.-%, bezogen auf das Gesamtgewicht der Tablette, zu der in Schritt (a) (ii.) gebildeten Mischung und Mischen; und
(b) Bilden der dispergierbaren Tablette durch Verpressen der in Schritt (a) (iii.) erhaltenen Mischung, mit der Maßgabe, dass es sich bei dem Verdünnungsmittel gemäß Schritt (a) (i.) oder (a) (ii.) nicht um Calciumphosphat, wasserfreies Calciumhydrogenphosphat oder Calciumhydrogenphosphatdihydrat handelt.

## Revendications

1. Comprimé dispersible comprenant (a) du 2-amide 1-({4-méthyl-5-[2-(2,2,2-trifluoro-1,1-diméthyl-éthyl)-pyridin-4-yl]-thiazol-2-yl}-amide) d'acide (S)-pyrrolidine-1,2-dicarboxylique, ou un sel pharmaceutiquement acceptable correspondant, en une quantité de 5 % à 50 % en poids sur la base du poids total du comprimé, (b) un glycolate d'amidon de sodium, en une quantité de 2 % à 12 % en poids sur la base du poids total du comprimé, (c) une hydroxypropyle cellulose faiblement substituée, en une quantité de 1 % à 10 % en poids sur la base du poids total du comprimé, (d) du fumarate de stéaryle et de sodium, en une quantité de 1 % à 7 % en poids sur la base du poids total du comprimé, et éventuellement (e) un ou plusieurs excipients pharmaceutiquement acceptables supplémentaires, à la condition que ledit excipient pharmaceutiquement acceptable supplémentaire ne soit pas du phosphate de calcium tribasique, du phosphate de calcium dibasique anhydre, du dihydrate de phosphate de calcium dibasique ou du sucralose.

2. Comprimé dispersible selon la revendication 1, comprenant des granulés ayant une phase interne et une phase externe, dans lequel la phase interne comprend le 2-amide 1-({4-méthyl-5-[2-(2,2,2-trifluoro-1,1-diméthyl-éthyl)-pyridin-4-yl]-thiazol-2-yl}-amide) d'acide (S)-pyrrolidine-1,2-dicarboxylique ou un sel pharmaceutiquement acceptable correspondant, le glycolate d'amidon de sodium, l'hydroxypropyle cellulose faiblement substituée, et éventuellement de quelconques éventuels excipients pharmaceutiquement acceptables supplémentaires, dans lequel la phase externe comprend du glycolate d'amidon de sodium, du fumarate de stéaryle et de sodium et facultativement d'éventuels quelconques excipients pharmaceutiquement acceptables supplémentaires, à condition que ledit excipient pharmaceutiquement acceptable supplémentaire dans la phase interne ou la phase externe ne soit pas du phosphate de calcium tribasique, du phosphate de calcium dibasique anhydre, du dihydrate de phosphate de calcium dibasique ou du sucralose.

3. Comprimé dispersible selon l'une quelconque des revendications précédentes, dans lequel le glycolate d'amidon de sodium est présent en une quantité totale de 5 % à 12 %, par exemple, 5 à 10 % en poids sur la base du poids total du comprimé.

4. Comprimé dispersible selon l'une quelconque des revendications précédentes, dans lequel l'hydroxypropyle cellulose faiblement substituée est présente en une quantité totale de 2 % à 5 %, par exemple 1 % à 3 %, en poids sur la base du poids total du comprimé.

5. Comprimé dispersible selon l'une quelconque des revendications précédentes, dans lequel le fumarate de stéaryle et de sodium est présent en une quantité totale de 3 % à 6 %, par exemple, 3 % à 5 % en poids sur la base du poids total du comprimé.

6. Comprimé dispersible selon l'une quelconque des revendications précédentes, dans lequel le comprimé dispersible comprend en outre un excipient pharmaceutiquement acceptable supplémentaire qui est un diluant, à condition que le diluant ne soit pas du phosphate de calcium tribasique, du phosphate de calcium dibasique anhydre ou du dihydrate de phosphate de calcium dibasique.

7. Comprimé dispersible selon la revendication 6, dans lequel le diluant est choisi parmi le mannitol (y compris les polymorphes alpha, bêta et delta), le sorbitol, la maltodextrine, le lactose (par exemple, le lactose monohydraté), une cellulose microcristalline, le maltitol, le xylitol, un amidon (par ex. maïs ou riz) ou toute combinaison correspondante.

8. Comprimé dispersible selon la revendication 7, dans lequel la quantité totale de diluant est dans la plage de 20 % à 70 %, par exemple 30 % à 60 %, par exemple 35 % à 55 %, par exemple, 45 % à 55 %, par exemple, 45 % à 50 % en poids, sur la base du poids total du comprimé.

9. Comprimé dispersible selon l'une quelconque des revendications précédentes, dans lequel le comprimé a un temps de désintégration de 5 minutes ou moins, de préférence de 3 minutes ou moins, lorsqu'il est mesuré dans l'eau à une température de 15 à 25 °C selon l'essai normalisé pour des comprimés dispersibles décrit dans la huitième édition de la Pharmacopée européenne, supplément 8.2, page 811, janvier 2014, en combinaison avec la Pharmacopée européenne, huitième édition, supplément 8.2, pages 285 à 287, section 2.9.1, janvier 2014.

10. Comprimé dispersible selon l'une quelconque des revendications précédentes, pour une utilisation dans le traitement ou la prévention d'une maladie proliférative, de préférence un cancer.

11. Procédé pour la production d'un comprimé dispersible selon la revendication 1, comprenant
(a) la formation d'un granulé ayant une phase interne et une phase externe par
(i.) granulation humide d'une phase interne comprenant du 2-amide 1-({4-méthyl-5-[2-(2,2,2-trifluoro-1,1-diméthyl-éthyl)-pyridin-4-yl]-thiazol-2-yl}-amide) d'acide (S)-pyrrolidine-1,2-dicarboxylique ou un sel pharmaceutiquement acceptable correspondant en une quantité de 5 à 50 % en poids sur la base du poids total du comprimé, du glycolate d'amidon de sodium en une quantité totale de 1 % à 7 % en poids sur la base du poids total du comprimé, une hydroxypropyle cellulose faiblement substituée, en une quantité de 1 % à 10 % en poids sur la base du poids total du comprimé, et au moins un diluant ;
(ii.) ajout de glycolate d'amidon de sodium en une quantité totale de 1 % à 5 % en poids sur la base du poids total du comprimé, et d'au moins un diluant dans la phase interne formée à l'étape (a) (i.) et mélange ; et
(iii.) ajout de fumarate de stéaryle et de sodium en une quantité totale de 1 % à 7 % en poids par rapport au poids total du comprimé au mélange formé à l'étape (a) (ii.) et mélange ; et
(b) formation du comprimé dispersible par compression du mélange obtenu à l'étape (a) (iii.),
à condition que le diluant selon l'étape (a)(i.) ou (a) (ii.) ne soit pas du phosphate de calcium tribasique, du phosphate de calcium dibasique anhydre ou du dihydrate de phosphate de calcium dibasique.
